(19) Europäisches Patentamt
European Patent Office
Office européen des brevets

(11) Veröffentlichungsnummer: **0 229 890**
**B1**

(12) # EUROPÄISCHE PATENTSCHRIFT

(45) Veröffentlichungstag der Patentschrift: **22.02.89**

(51) Int. Cl.[4]: **C07C 143/44**, C07C 143/46, C07C 139/00

(21) Anmeldenummer: **86114543.1**

(22) Anmeldetag: **21.10.86**

(54) **Verfahren zur Herstellung von Acyloxibenzolsulfonsäuren und deren Salzen.**

(30) Priorität: **26.10.85 DE 3538143**

(43) Veröffentlichungstag der Anmeldung:
**29.07.87 Patentblatt 87/31**

(45) Bekanntmachung des Hinweises auf die Patenterteilung:
**22.02.89 Patentblatt 89/8**

(84) Benannte Vertragsstaaten:
**AT BE CH DE ES FR GB IT LI NL SE**

(56) Entgegenhaltungen:
**US-A- 3 503 888**

**CHEMICAL ABSTRACTS, Band 91, nr. 13, 24. September 1979, Seite 565, Zusammenfassung nr. 107806y, Columbus, Ohio, US; & PL-A-99 682 (INSTYTUT CIEZKIEJ SYNTEZY ORGANICZNEJ "BLACHOWNIA") 15-11-78**

(73) Patentinhaber: **HOECHST AKTIENGESELLSCHAFT, Postfach 80 03 20, D-6230 Frankfurt am Main 80(DE)**

(72) Erfinder: **Grabley, Fritz-Feo, Dr., Hölderlinstrasse 7, D-6240 Königstein/Taunus(DE)**
Erfinder: **Reinhardt, Gerd, Dr., Freiherr-vom-Stein-Strasse 37, D-6233 Kelkheim (Taunus)(DE)**
Erfinder: **Bäder, Georg, Dr., Brandenburger Weg 26, D-6238 Hofheim am Taunus(DE)**
Erfinder: **Rupp, Walter, Dr., Am Eichkopf 6, D-6240 Königstein/Taunus(DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 229 890 B1

ACTORUM AG

**Beschreibung**

Die Erfindung betrifft die Herstellung von Acyloxibenzolsulfonsäuren und deren Salzen in einer mehrstufigen Reaktionsfolge ausgehend von Phenol und Schwefelsäure oder Oleum.

Acyloxibenzolsulfonsäuren und ihre Salze sind seit langem bekannte Verbindungen mit tensidischen Eigenschaften, die zudem gemäß EP 98 021 als Perborataktivatoren Verwendung finden können. Zu ihrer Herstellung sind bereits eine Reihe von Verfahren bekannt.

Sie können beispielsweise durch Umsetzung von Na-Phenolsulfonat mit Säurechloriden (EP 98 129), mit Anhydriden (EP 105 673) oder Estern (EP 125 641) erhalten werden. Die Reaktionen verlaufen sehr langsam und müssen normalerweise bei hoher Temperatur durchgeführt werden. Zudem ist die Herstellung von wasserfreiem Na-Phenolsulfonat sehr kostenintensiv. Nach DE 666 626 werden Acyloxibenzolsulfonsäuren durch Sulfierung von Phenolestern erhalten.

Als ökonomischstes Verfahren ist jedoch das in DE-A 3 524 052 beschriebene Verfahren anzusehen. Dieses Verfahren verläuft in vier Stufen, nämlich

a) Sulfierung von Phenol mit Chlorsulfonsäure oder $SO_3$,

b) Temperung der Phenolsulfonsäure zur Erhöhung des Anteils des 4-Isomeren,

c) Veresterung der Phenolsulfonsäure mit einem Derivat einer organischen Säure (Säurechlorid oder Anhydrid) oder einer organischen Säure in Gegenwart wasserentziehender Mittel (z.B. $SOCl_2$),

d) pH-kontrollierte Neutralisation des Esters und ggf. Isolierung des Salzes z.B. durch Sprühtrocknung.

Bei diesem Verfahren wird, im Unterschied zu dem im folgenden beschriebenen, erfindungsgemäßen Verfahren, die Sulfierung mit Chlorsulfonsäure oder $SO_3$ vorgenommen um wasserfreie Phenolsulfonsäure zu erhalten.

Die Verwendung wasserfreier Phenolsulfonsäure ist besonders wichtig für die oben beschriebene mehrstufige Reaktionsfolge zur Herstellung von Acyloxibenzolsulfonsäuren, da bei Anwesenheit von Wasser die in Stufe c) gebildete Acyloxibenzolsulfonsäure leicht hydrolysiert, was zu einer drastischen Ausbeuteminderung führt.

Aus dem polnischen Patent 99 682 (CA 91, 107, 806) ist bekannt, daß wasserfreie Phenolsulfonsäure aus Phenol und Schwefelsäure erhalten werden kann, wenn das bei der Reaktion gebildete Reaktionswasser aus dem Reaktionsmedium entfernt wird. Dies geschieht dadurch, daß man Phenol mit $H_2SO_4$ sulfiert und das Sulfiergemisch durch eine Heizmantelkolonne getropft wird, in der ein entgegenkommender Gasstrom eines inerten organischen Lösungsmittels, z.B. $C_7H_{16}$, das Reaktionswasser azeotrop entfernt. Nachteile dieses Verfahrens sind der apparative Aufwand sowie die aufwendige Abtrennung des organischen Lösemittels vom Reaktionsprodukt. Nach Ind. eng. Chem. 32, 408 (1940) kann das Reaktionswasser auch durch Zusatz von $BF_3$ zum Reaktionsgemisch als $BF_3 \cdot 2\,H_2O$ destillativ entfernt werden, dabei muß das $BF_3$ jedoch in einer nachgeschalteten Stufe über $Ca(BF_4)_2$ wiedergewonnen werden.

Aufgabe der vorliegenden Erfindung ist es, ein auch im großtechnischen Maßstab leicht durchführbares Verfahren zur Herstellung von Acyloxibenzolsulfonsäuren oder deren Salzen zu entwickeln, wobei in der Stufe a) Phenolsulfonsäure aus Phenol und Schwefelsäure bzw. Oleum erhalten wird, die auf einer späteren Stufe entwässert wird.

Gegenstand der Erfindung ist ein Verfahren zur Herstellung von Acyloxibenzolsulfonsäuren und deren Salzen der Formel

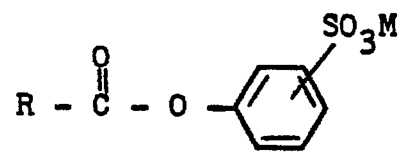

wobei R $C_1$–$C_{17}$-Alkyl oder $C_2$–$C_{17}$-Alkenyl und M ein Alkali- oder Erdalkalimetall, Ammoniumion oder Wasserstoff bedeuten mit den Stufen (a) Sulfierung von Phenol, gegebenenfalls (b) thermische Behandlung des sulfierten Phenols, (c) Veresterung des sulfierten Phenols und gegebenenfalls (d) Neutralisierung des Esters, wobei man die Sulfierung des Phenols mit Schwefelsäure oder Oleum durchführt und während der Temperung oder im Anschluß daran das Reaktionswasser durch Destillation und/oder durch Reaktion mit einem wasserentziehenden Mittel entfernt.

Die Sulfierung des Phenols wird durchgeführt mit 50 bis 100%iger, vorzugsweise 96 bis 98%iger Schwefelsäure oder mit Oleum bevorzugt mit einem Gehalt an $SO_3$ von 20% oder 65%. Die Reaktionstemperatur liegt bei Verwendung von Schwefelsäure zwischen 30 und 150°C, bevorzugt zwischen 50 und 110°C. Bei Verwendung von Oleum liegt die Reaktionstemperatur zwischen 30 und 110°C, vorzugsweise zwischen 30 und 60°C. Die Menge an Schwefelsäure oder Oleum für die Sulfierung wird so gewählt, daß auf 1 Mol Phenol 0,8 bis 1,2 Mol, vorzugsweise 0,95 bis 1,1 Mol an Sulfierungsreagenz vorhanden sind. Die Sulfierungsreaktion kann in Abwesenheit eines Lösemittels oder in Gegenwart von 5 bis 120 Mol-% (bezogen auf Phenol) einer $C_2$–$C_{18}$-Alkansäure, z.B. 3,5,5-Trimethylhexansäure, erfolgen. Durch die Anwesenheit dieser Säure wird die nachfolgende Abdestillation des Wassers erheblich beschleunigt.

Die weitere Umsetzung der so erhaltenen Phenolsulfonsäure zur Acyloxibenzolsulfonsäure erfolgt wie in DE-A 3 524 052 beschrieben. Je nach Zusammensetzung des Phenolsulfonsäuregemisches erfolgt ggf. eine Temperung der Phenolsulfonsäure. Bei dieser Temperung wird die Phenolsulfonsäure thermisch so behandelt, daß der Anteil der 4-Phenolsulfonsäure relativ zum Anteil der 2-Phenolsulfonsäure und damit auch in absoluter Menge zunimmt. Dazu wird die gebildete Phenolsulfonsäure auf eine Temperatur von insbesondere 50 bis 110°C

erhitzt. Der Anteil des 4-Isomeren steigt bei dieser Temperung von (bezogen auf den Mengenanteil des 2-Isomeren) etwa 2 bis 3 zu 1 ohne Temperungsstufe auf bevorzugt etwa 8 bis 20 zu 1 (je nach Temperungsbedingungen). Die Temperungszeit beträgt im allgemeinen 0,5 bis 10 h. Diese Temperung kann aber auch entfallen, besonders dann, wenn das Phenolsulfonsäuregemisch bereits von vornherein das gewünschte Verhältnis der 2- und 4-Isomeren aufweist.

Im Anschluß daran oder während der Temperstufe wird das entstandene Reaktionswasser entweder durch Destillation oder durch Zugabe eines wasserentziehenden Mittels entfernt. Man kann aber auch beide Methoden gemeinsam anwenden, wobei dann zunächst das Reaktionswasser weitgehend destillativ entfernt wird und anschließend eine Nachbehandlung des Reaktionsgemisches mit einem wasserentziehenden Mittel erfolgt. Die Destillation erfolgt bei Normaldruck oder unter vermindertem Druck und kann, ebenso wie die Sulfierung, kontinuierlich oder diskontinuierlich gestaltet werden. Dabei wird ein Restwassergehalt von höchstens 5 Gew.-% angestrebt.

Wird die Sulfierung mit 65 proz. Oleum durchgeführt, so wird eine weitere Reduzierung des Wassergehalts durch Zugabe von 0,3 bis 0,5 Mol eines wasserentziehenden Mittels, wie zum Beispiel $SOCl_2$ oder mit 0,1 bis 0,25 Mol $POCl_3$, $PCl_3$, $P_4O_{10}$ oder ähnliche Verbindungen erreicht. Bei Verwendung von Schwefelsäure richtet sich die Menge des wasserentziehenden Mittels nach dem Restwassergehalt in der Phenolsulfonsäure. Um eine ausreichende Reaktion dieser wasserentziehenden Mittel mit dem Restwasser zu gewährleisten, erfolgt dieser Schritt bei Temperaturen von 30 bis 120°C und einer Reaktionszeit von 15 Minuten bis 6 Stunden, vorzugsweise 30 Minuten bis 2 Stunden. Die Temperatur bei Verwendung von Thionylchlorid liegt vorzugsweise bei 30 bis 60°C und für $P_4O_{10}$ bei 60 bis 90°C. Falls die bei dieser Temperatur entstandenen Hydrolyseprodukte gasförmig sind, können sie mit Hilfe eines Inertgases oder durch verminderten Druck aus dem Sulfiergemisch entfernt werden. Falls es sich um feste oder flüssige Nebenprodukte handelt, können sie im Sulfiergemisch verbleiben, so daß dann, beispielsweise bei Verwendung von wasserentziehenden Phosphorverbindungen, das Endprodukt nach der Stufe (d) $NaH_2PO_4$ oder $Na_2H_2P_2O_7$ enthält.

Die Kombination beider Verfahrensschritte, d.h. zunächst Abdestillation des größten Teils des Reaktionswassers und anschließende Bindung des Restwassers durch Zugabe eines wasserentziehenden Mittels ist besonders vorteilhaft für den Fall, daß die Sulfierung mit Schwefelsäure erfolgt. Bei der Sulfierung mit Oleum ist es meist ausreichend, das Reaktionswasser durch Zugabe eines wasserentziehenden Mittels zu entfernen.

In der sich anschließenden Acylierungsstufe (c) wird die wasserfreie Phenolsulfonsäure verestert, wobei die Phenolsulfonsäuren der Stufen (a) oder (b) mit ($C_2$–$C_{18}$)Alkansäurehalogeniden (insbesondere -chloriden) oder -anhydriden ohne Zusätze oder mit den freien Säuren oder ihren Anhydriden in Gegenwart wasserentziehender Mittel wie $SOCl_2$ oder $POCl_3$ umgesetzt werden. Diese Stufe kann in Anwesenheit von aprotischen organischen Lösemitteln durchgeführt werden, aber insbesondere auch eine lösemittelfreie Variante führt zum Erfolg. Die Reaktionstemperatur liegt im allgemeinen bei 0° bis 110°C, vorzugsweise bei 20 bis 80°C, die Reaktionszeit bei 0,5 bis 8 h. Die eingesetzte Menge an Acylierungsmittel beträgt bevorzugt 0,8 bis 1,2, insbesondere 0,9 bis 1,1 Mol pro Mol Sulfonsäure; wenn Anhydride zum Einsatz kommen reduziert sich diese Menge auf 0,4 bis 0,6 Mol. Das eingesetzte wasserentziehende Mittel wird in der Regel in geringem Überschuß zugegeben. Da bei der Veresterung gelegentlich mit dem Auftreten von Schaum gerechnet werden muß, empfiehlt sich in diesen Fällen der Zusatz eines Entschäumers, beispielsweise auf Silikonbasis. Der Veresterungsstufe kann sich eine Nachbehandlung mit $SOCl_2$ oder $POCl_3$ anschließen. Als geeignete Acylierungsmittel werden die sich von $C_6$–$C_{18}$-Alkansäure ableitenden Verbindungen bevorzugt, z.B. von Isononansäure (3,5,5-Trimethylhexansäure), Nonansäure, 2-Ethyl-hexansäure, Dodecansäure, Hexadecansäure und Octadecansäure. Die Kohlenwasserstoffreste dieser Verbindungen können gesättigt oder ungesättigt und geradkettig oder verzweigt sein; die Carboxylgruppe bzw. eine sich davon ableitende funktionelle Gruppe kann auch als Substituent innerhalb des Kohlenwasserstoffrestes stehen, bevorzugt ist sie jedoch endständig. In der Praxis sind die Alkansäuren häufig Gemische von Verbindungen unterschiedlicher Kettenlänge oder sie enthalten mehr oder weniger große Anteile an ungesättigten Verbindungen, im Mittel sollen sie aber in den vorstehenden Bereich fallen.

Der Acylierungsstufe (c) schließt sich gegebenenfalls – sofern die Herstellung der Salze beabsichtigt ist – noch eine Neutralisationsstufe (d) an, bei der die Sulfonsäuregruppe in die Salzform überführt wird. Die Neutralisation – vorzugsweise in wäßrigem Medium – wird bei kontrollierten pH-Werten durchgeführt, um eine Hydrolyse der in Stufe (c) gebildeten Acyloxybenzolsulfonsäuren möglichst weitgehend zu verhindern; sie kann im Bereich von pH 2 bis 7,5 gefahren werden, bevorzugt wird jedoch ein Bereich von 3 bis 6. Die Temperatur beträgt in dieser Stufe zweckmäßig 0 bis 50°C und als Basen werden beispielsweise Alkalimetall-, Erdalkalimetall- oder Ammoniumhydroxide, -carbonate oder -hydrogencarbonate eingesetzt.

Das nach Beendigung der Stufe (d) erhaltene Produkt wird abschließend getrocknet, beispielsweise durch Sprühtrocknung. Das erfindungsgemäße Verfahren kann diskontinuierlich, aber auch kontinuierlich in auf diesem Arbeitsgebiet üblichen Apparaten durchgeführt werden.

Das erfindungsgemäße Verfahren weist gegenüber dem Stand der Technik insbesondere folgende Vorteile auf:

– Die Sulfierung ist auch im großtechnischen Maßstab durch den Einsatz von $H_2SO_4$ bzw. Oleum apparativ einfacher durchzuführen als bei Verwendung von $ClSO_3H$ oder $SO_3$.

– Durch das bei der Sulfierung im Reaktionsgemisch entstehende Wasser wird die Bildung von Nebenprodukten (z.B. Sulfonen oder Sulfonsäureestern) deutlich unterdrückt, so daß eine qualitativ bessere Acyloxibenzolsulfonsäure erhalten wird.

In den folgenden Beispielen verhalten sich Gew.-Teile zu Vol.-Teilen wie kg zu $dm^3$, %-Angaben beziehen sich – wenn nichts anderes angegeben ist – immer auf das Gewicht. Unter dem Begriff WS-Gehalt ist in den folgenden Beispielen der Anteil an Wirksubstanz im Produkt zu verstehen, der durch 2-Phasen-Titration nach EPTON bestimmt wird.

Vergleichsbeispiel V 1

Das Beispiel beschreibt die Herstellung und Umsetzung von Phenolsulfonsäure aus Phenol und Oleum ohne Entfernung des Reaktionswassers.

94 Gew.-Teile Phenol werden bei einer Temperatur von 40 bis 45°C geschmolzen. Unter Kühlung werden dann 89,7 Gew.-Teile 65%iges Oleum so zugetropft, daß die Reaktionstemperatur 50°C nicht übersteigt, danach wird 2 Stunden bei 80°C nachgerührt.

Bei einer Temperatur von 45 bis 50°C werden dann 182 Gew.-Teile Isononansäurechlorid innerhalb 1 Stunde zu dem Sulfiergemisch getropft, und das Reaktionsgemisch wird 1 Stunde nachgerührt; während und nach Beendigung dieser Stufe (c) wird darauf geachtet, daß das entstehende HCl-Gas ausgetragen wird. Das praktisch in quantitativer Ausbeute erhaltene Reaktionsprodukt weist einen WS-Gehalt von 68% auf; naoh 6-stündigem Stehen bei 25°C weist das Reaktionsprodukt nur noch einen Gehalt von 52% auf.

Beispiel 1

Es wird nach den Angaben des Vergleichsbeispiels V 1 verfahren, aber vor der Veresterung 40 Gew.-Teile Thionylchlorid zugegeben und das Gemisch 1 Stunde bei 80°C nachgerührt. Die Isononanoyloxibenzolsulfonsäure weist einen WS-Gehalt von 81,9% auf, der über mehrere Tage stabil ist. Das Na-Salz als Endprodukt weist einen WS-Gehalt von 83% auf.

Beispiel 2

Es wird nach den Angaben des Vergleichsbeispiels V 1 verfahren, aber vor der Veresterung 15,8 Gew.-Teile $P_2O_5$ zugegeben und das Gemisch 1 Stunde bei 80°C nachgerührt. Die Isononanoyloxibenzolsulfonsäure weist einen stabilen WS-Gehalt von 79,2%, das Na-Salz als Endprodukt einen WS-Gehalt von 80% auf.

Beispiel 3

Es werden 94 Gew.-Teile Phenol in 163 Gew.-Teilen Isononansäure gelöst. Unter Kühlung werden 89,7 Gew.-Teile 65%iges Oleum so zugetropft, daß die Reaktionstemperatur 50°C nicht übersteigt, danach wird 2 Stunden bei 80°C nachgerührt. Bei 45 bis 50°C werden dann 166 Gew.-Teile Thionylchlorid innerhalb 1 Stunde zugetropft und 1 Stunde nachgerührt. Die Isononanoyloxibenzolsulfonsäure weist einen stabilen WS-Gehalt von 81%, das praktisch in quantitativer Ausbeute erhaltene Na-Salz als Endprodukt weist einen WS-Gehalt von 84% auf.

Vergleichsbeispiel V 2

Das Beispiel beschreibt die Herstellung und Umsetzung von Phenolsulfonsäure, die aus Phenol und Schwefelsäure ohne Entfernung des Reaktionswassers erhalten wird.

Es werden zu 94 Gew.-Teilen Phenol bei einer Temperatur zwischen 40 und 50°C 107 Gew.-Teile 96%ige Schwefelsäure innerhalb 30 Minuten zugetropft. Anschließend wird 2 Stunden bei 80°C nachgerührt, dann werden bei 40 bis 45°C 182 Gew.-Teile Isononansäurechlorid innerhalb 1 Stunde zugetropft und 1 Stunde nachgerührt. Man erhält eine Isononanoyloxibenzolsulfonsäure mit einem WS-Gehalt von 40%, der beim Stehen bei 25°C weiter absinkt.

Beispiel 4

94 Gew.-Teile Phenol werden in 166 Gew.-Teilen Isononansäure gelöst. Zu dieser Lösung werden dann 107 Gew.-Teile 96%ige Schwefelsäure gegeben und das Gemisch 30 Minuten auf 100 bis 110°C erhitzt. Anschließend wird das Reaktionswasser unter reduziertem Druck (20 mm) innerhalb 30 Minuten weitgehend abdestilliert. Nach dem Abkühlen werden bei 45 bis 50°C 134 Gew.-Teile Thionylchlorid unter Zusatz eines Entschäumers innerhalb von 30 Minuten zugetropft. Es wird 1 Stunde nachgerührt. Man erhält eine stabile Isononanoyloxibenzolsulfonsäure mit einem WS-Gehalt von 81%, das Na-Salz als Endprodukt, das in praktisch quantitativer Ausbeute (bez. auf Phenol) erhalten wird, weist einen WS-Gehalt von 85% auf.

Beispiel 5

94 Gew.-Teile Phenol werden geschmolzen vorgelegt und bei einer Temperatur bis 80°C mit 103 Gew.-Teilen 96%iger Schwefelsäure in 30 Minuten sulfoniert. Anschließend wird das Reaktionswasser unter reduziertem Druck (20 mm) in 3 Stunden bei 105°C weitgehend abdestilliert. Bei 80°C werden dann 16,7 Gew.-Teile $P_2O_5$ eingetragen und 30 Minuten bei 85°C nachgerührt. Nach dem Abkühlen auf 45 bis 50°C werden 188 Gew.-Teile Isononansäurechlorid innerhalb von 30 Minuten zugetropft und 30 Minuten nachgerührt. Man erhält eine Isononanoyloxibenzolsulfonsäure mit einem WS-Gehalt von 79%, aus der nach Neutralisation ein Na-Salz mit einem WS-Gehalt von 83% isoliert wird.

Beispiel 6

Es wird zunächst nach Beispiel 5 verfahren. Nach Abdestillieren von Wasser unter vermindertem Druck werden 54,6 Gew.-Teile Thionylchlorid zugesetzt und 30 Minuten bei 80°C nachgerührt. Anschließend wird weiter nach Beispiel 5 verfahren. Man erhält eine Isononanoyloxibenzolsulfonsäure mit einem WS-Gehalt von 80% und ein Na-Salz mit einem WS-Gehalt von 83%.

**Patentansprüche**

1. Verfahren zur Herstellung von Acyloxibenzolsulfonsäuren und deren Salzen der Formel

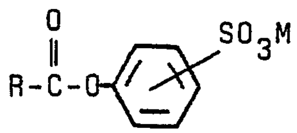

wobei R $C_1$–$C_{17}$-Alkyl oder $C_2$–$C_{17}$-Alkenyl und M ein Alkali- oder Erdalkalimetall, Ammonium oder Wasserstoff bedeuten mit den Stufen
(a) Sulfierung von Phenol, gegebenenfalls (b) thermische Behandlung des sulfierten Phenols, (c) Veresterung des sulfierten Phenols und gegebenenfalls (d) Neutralisierung des Esters, dadurch gekennzeichnet, daß man die Sulfierung des Phenols mit 50 bis 100%iger Schwefelsäure oder mit Oleum bei Temperaturen von 30 bis 150°C durchführt, wobei auf 1 Mol Phenol 0,8 bis 1,2 Mol Sulfierungsreagenz vorhanden ist, und während der thermischen Behandlung oder im Anschluß daran das Reaktionswasser durch Destillation und/oder durch Reaktion mit einem wasserentziehenden Mittel entfernt.

2. Verfahren nach Anspruch 1, dadurch gekennzeichnet, daß man die Sulfierungsreaktion in Gegenwart von 5 bis 120 Mol-% einer $C_2$–$C_{18}$-Alkansäure durchführt.

3. Verfahren nach Anspruch 1 oder 2, dadurch gekennzeichnet, daß man als wasserentziehendes Mittel Thionylchlorid, Phosphoroxichlorid, Phosphortrichlorid oder Phosphorpentoxid verwendet.

**Claims**

1. A process for the preparation of an acyloxybenzenesulfonic acid and salts thereof, of the formula

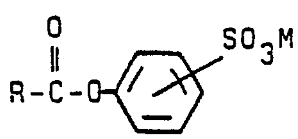

in which R is $C_1$–$C_{17}$-alkyl or $C_2$–$C_{17}$-alkenyl and M is an alkali or alkaline earth metal, ammonium or hydrogen, including the stages of (a) sulfonation of phenol, if appropriate (b) thermal treatment of the sulfonated phenol, (c) esterification of the sulfonated phenol and if appropriate (d) neutralization of the ester, which comprises carrying out the sulfonation of the phenol with 50 to 100% sulfuric acid or with oleum at temperatures from 30 to 150°C, 0.8 to 1.2 mol of sulfonation reagent being present for 1 mol of phenol, and removing the water of reaction during or after the thermal treatment by distillation and/or by reaction with a dehydrating agent.

2. The process as claimed in claim 1, wherein the sulfonation reaction is carried out in the presence of 5 to 120 mol% of a $C_2$–$C_{18}$-alkanoic acid.

3. The process as claimed in claim 1 or 2, wherein the dehydrating agent used is thionyl chloride, phosphorus oxychloride, phosphorus trichloride or phosphorus pentoxide.

**Revendications**

1. Procédé de préparation d'acides acyloxybenzènesulfoniques et de leurs sels, de formule:

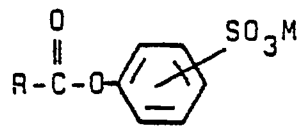

dans laquelle R désigne un alkyle en $C_1$–$C_{17}$ ou un alcényle en $C_2$–$C_{17}$ et M un métal alcalin ou alcalinoterreux ou bien l'ion ammonium ou l'hydrogène par les étapes:
(a) sulfonation du phénol, éventuellement (b) traitement à chaud du phénol sulfoné formé, (c) estérification du phénol sulfoné et le cas échéant (d) neutralisation de l'ester, procédé caractérisé en ce que l'on effectue la sulfonation du phénol avec de l'acide sulfurique à une concentration de 50 à 100% ou avec de l'oléum, à des températures de 30 à 150°C, avec de 0,8 à 1,2 mol du réactif de sulfonation par mol de phénol, et au cours du traitement à chaud du phénol sulfoné ou après ce traitement on élimine l'eau formée par la réaction par distillation et/ou par réaction avec un agent déshydratant.

2. Procédé selon la revendication 1 caractérisé en ce que l'on effectue la réaction de sulfonation en présence de 5 à 120 Mol-% d'un acide alcanoïque en $C_2$–$C_{18}$.

3. Procédé selon la revendication 1 ou 2, caractérisé en ce que l'on utilise comme déshydratant le chlorure de thionyle, l'oxychlorure de phosphore, le trichlorure de phosphore ou le pentoxyde de phosphore.